# EUROPEAN PATENT APPLICATION

(11) **EP 1 688 743 A1**
(43) Date of publication of application: **09.08.2006**
(21) Application number: 04792934.4
(22) Date of filing: 25.10.2004
(51) Int. Cl.: G01N 33/543, G01N 21/78

(54) **READER FOR IMMUNOCHROMATOGRAPHIC TEST, CARTRIDGE APPLICABLE TO THIS, AND SYSTEM FOR EXAMINING IMMUNOCHROMATOGRAPHIC TEST PIECE**

(30) Priority: 29.10.2003 JP 2003369384
(71) Applicant: HAMAMATSU PHOTONICS K. K., Hamamatsu-shi, Shizuoka 435-8558 (JP)
(72) Inventor: NISHIKAWA, Masataka, c/o Hamamatsu Photonics K.K.,, Hamamatsu-shi, Shizuoka 4358-558 (JP); KOIKE, Takashi, c/o Hamamatsu Photonics K.K.,, Hamamatsu-shi, Shizuoka 435-8558 (JP)
(74) Representative: Grünecker, Kinkeldey, Stockmair & Schwanhäusser Anwaltssozietät
(86) International application number: PCT/JP2004/015806
(87) International publication number: WO 2005/040801

(57) **Abstract**

The present invention relates to an immunochromatographic test reader, etc., having structures that enable accommodation of a plurality of immunochromatographic test utensils that differ in outer shape and enable immunochromatographic test utensils to be managed readily in association with information concerning the immunochromatographic test utensils. The reader has a structure that illuminates measuring light onto an immunochromatographic test strip included in an immunochromatographic test utensil and that detects light arising from the immunochromatographic test strip as a result of the illumination of the measuring light, and a cartridge, with a structure that can be detachably attached to the reader, comprises: a holding section for holding the immunochromatographic test utensil that includes the immunochromatographic test strip; information storage means for storing information concerning the immunochromatographic test utensil; and a structure for introducing the immunochromatographic test utensil to a position where the measuring light is illuminated onto the immunochromatographic test strip.

## Description

### Technical Field

The present invention relates to an immunochromatographic test reader, a cartridge applicable to the reader, and an immunochromatographic test strip inspection system.

### Background Art

An immunochromatographic test strip has antibodies (or antigens), which undergo antigen-antibody reactions with antigens (or antibodies) in a sample, coated in band-like manner on a specific position of the immunochromatographic test strip. When a dye-labeled antigen (or antibody) in the sample is developed to the specific position by a developing solution, the antigen (or antibody) in the sample undergoes an antigen-antibody reaction with an antibody (or antigen) that has been coated in band-like manner and becomes trapped, and a colored region (for example, a colored line) due to the dye is formed at the specific position. By optically measuring the degree of coloration of the colored region formed in the immunochromatographic test strip, the amount of the antigen (or antibody) in the sample can be analyzed quantitatively.

In a reader that measures the degrees of coloration of an immunochromatographic test strip, an immunochromatographic test utensil, which includes the immunochromatographic test strip and a casing that holds the immunochromatographic test strip, is inserted from an opening portion of the main body of the reader. The reader then illuminates the immunochromatographic test strip with measuring light and detects light reflected from the immunochromatographic test strip as a result of the illumination of the measuring light (see for example, Patent Document 1).

In the reader described in Patent Document 1, a tray, on which the immunochromatographic test utensil is placed, is moved with respect to a housing provided with a light emitting means (LED, etc.) and a light receiving means (photodiode, etc.), and during this movement, the measuring light is illuminated from the light emitting means and the reflected light is detected by the light receiving means.

### Patent Document 1: Japanese Patent Application Laid-Open No. H11-83745

### Disclosure of the Invention

### Problem to be Solved by the Invention

The outer shapes of immunochromatographic test utensils differ, for example, in size according to the type of inspection, and thus numerous immunochromatographic test utensils of different outer shapes exist.

The inventors have studied conventional readers in detail, and as a result, have found problems as follows. Namely, the differences of the outer shapes of immunochromatographic test utensils are not taken into consideration at all in conventional readers, and a reader thus has a shape that is specialized for an immunochromatographic test utensil of a single shape. It was thus difficult to accommodate immunochromatographic test utensils of different shapes, and in the worst cases, measurements could not be made.

Immunochromatographic test utensils also vary widely not only in outer shape but also in the shapes, positions, etc., of windows that expose the immunochromatographic test strip, and thus a reading method suited for the immunochromatographic test utensil used must be set. An immunochromatographic test utensil must thus be managed according to various information concerning the immunochromatographic test utensil, such as the sample to be inspected, the reading method suitable for an inspection subject or the immunochromatographic test utensil, etc.

The present invention has been made to resolve the above problems and an object thereof is to provide an immunochromatographic test reader, a cartridge applicable to the reader, and an immunochromatographic test strip inspection system having structures, which enable accommodation of a plurality of immunochromatographic test utensils that differ in outer shape and enable immunochromatographic test utensils to be managed readily in association with information concerning the immunochromatographic test utensils.

### Means for Solving Problem

A cartridge according to the present invention has a structure that enables it to be detachably attached to an immunochromatographic test reader. The reader has a structure that illuminates measuring light onto an immunochromatographic test strip included in an immunochromatographic test utensil and that detects light arising from the immunochromatographic test strip as a result of illumination of the measuring light. The cartridge applicable to such a reader comprises a holding section that holds the immunochromatographic test utensil, information storage means that stores information concerning the immunochromatographic test utensil, and a structure for introducing the immunochromatographic test utensil to a position where the measuring light is illuminated onto the immunochromatographic test strip included in the immunochromatographic test utensil.

In the cartridge according to the present invention, the immunochromatographic test utensil that is held in the cartridge is introduced to the position of the reader where the measuring light is illuminated onto the immunochromatographic test strip. Also, the cartridge holding the immunochromatographic test strip is readily removed from the reader after measurement. A plurality of immunochromatographic test utensils that differ in outer shape can thus be accommodated by the same reader by preparing, for example, a plurality of cartridges that respectively have holding sections of shapes corresponding to the immunochromatographic test utensils. Also, since the cartridge has information storage means, the immunochromatographic test utensil and the information concerning the immunochromatographic test utensil can be managed readily in associated form.

In the cartridge according to the present invention, the information concerning the immunochromatographic test utensil preferably includes information concerning an inspection that is performed using the immunochromatographic test utensil. In this case, the immunochromatographic test utensil and the information concerning the inspection using the immunochromatographic test utensil can be managed readily and accurately in associated form. Here, the information concerning the inspection using the immunochromatographic test utensil includes the name of an inspection subject, the date of inspection, the name of a person in charge of inspection, etc.

The information concerning the immunochromatographic test utensil may include information that identify the immunochromatographic test utensil. In this case, the immunochromatographic test utensil and the information that identify the immunochromatographic test utensil can be managed readily and accurately in associated form. Here, the information that identify the immunochromatographic test utensil includes the name of a manufacturer, the manufactured serial number, an item number, the name of an inspection item (for example, Salmonella inspection, endocrine disrupter inspection, influenza inspection, medical drug inspection, etc.), etc., of the immunochromatographic test utensil.

The information concerning the immunochromatographic test utensil may include information concerning a colored region reading process corresponding to the immunochromatographic test strip included in the immunochromatographic test utensil. In this case, the immunochromatographic test utensil and the information concerning the colored region reading process corresponding to the immunochromatographic test strip included in the immunochromatographic test utensil can be managed readily and accurately in associated form. Here, the information concerning the colored region reading process corresponding to the immunochromatographic test strip included in the immunochromatographic test utensil includes a waiting time until the start of scanning (reading), the number of scans, the number of colored lines, the positions of the colored lines, etc.

The immunochromatographic test utensil preferably includes a casing that holds the immunochromatographic test strip. In this case, the information concerning the immunochromatographic test utensil includes information concerning a colored region reading process corresponding to the casing included in the immunochromatographic test utensil. In this case, the immunochromatographic test utensil and the information concerning the colored region reading process corresponding to the casing included in the immunochromatographic test utensil can be managed readily and accurately in associated form. Here, the information concerning the colored region reading process corresponding to the casing includes the positions of windows for exposing the immunochromatographic test strip, the sizes of the windows, the existence of partitions in the windows, etc.

The information concerning the immunochromatographic test utensil may include information concerning a method of reading by the reader. In this case, the immunochromatographic test utensil and the information concerning the method of reading by the reader can be managed readily and accurately in associated form. Here, the information concerning the method of reading by the reader include the measurement precision, the scanning speed, the number of times of data accumulation, etc.

The information concerning the immunochromatographic test utensil may include information concerning methods of performing computations on data obtained by the reader. In this case, the immunochromatographic test utensil and the information concerning the methods of performing computations on the data obtained by the reader can be managed readily and accurately in associated form. Here, the information concerning the methods of performing computations on the data obtained by the reader include methods of performing computations on the data obtained by reading of colored regions (computation of average value, computation of peak value, computation of absorbance, etc.), methods of judging the data obtained by reading of the colored regions, scattering correction of coloration characteristics according to lot, etc.

The information concerning the immunochromatographic test utensil may include information concerning colored regions read by the reader. In this case, the immunochromatographic test utensil and the information concerning the colored regions read by the reader can be managed readily and accurately in associated form. Here, the information concerning the colored regions read by the reader includes data obtained by reading of the colored regions, judgment results based on the data, etc.

It is preferable that the cartridge according to the present invention further comprises an information sending means that sends the information concerning the immunochromatographic test utensil, stored in the information storage means, to the reader. By this arrangement, the information concerning the immunochromatographic test utensil that is stored in the information storage means can be sent to the reader.

The cartridge according to the present invention may further comprise a label for identifying the immunochromatographic test utensil. By this arrangement, the immunochromatographic test utensil that is held in the cartridge can be identified accurately by visual inspection by a person in charge of inspection, etc.

In the cartridge according to the present invention, the holding section is preferably a recess with a shape corresponding to the outer shape of the immunochromatographic test utensil including the immunochromatographic test strip. Also, the cartridge preferably has a first surface that faces the immunochromatographic test utensil that is held in the holding section, a second surface that opposes the first surface, and a through hole that puts the first surface and the second surface in communication at a portion of the holding section that overlaps with the immunochromatographic test utensil. By this arrangement, the taking out of the immunochromatographic test utensil held in the holding section is facilitated.

An immunochromatographic test reader according to the present invention comprises the cartridge (cartridge according to the present invention), with the above-described structure, and an information acquiring means that acquires information concerning the immunochromatographic test utensil that are stored in the information storage means provided in the cartridge.

In the immunochromatographic test reader according to the present invention with the above arrangement, the cartridge that holds the immunochromatographic test utensil is introduced to the position at which the measuring light is illuminated onto the immunochromatographic test strip. By this arrangement, colored regions formed on the immunochromatographic test strip, held by the immunochromatographic test utensil, can be read appropriately regardless of the outer shape of the immunochromatographic test utensil. Also, the information concerning the immunochromatographic test utensil that corresponds to the immunochromatographic test utensil that is held in the cartridge can be acquired, thereby enabling the immunochromatographic test utensil and the information concerning the immunochromatographic test utensil to be managed readily in associated form.

Furthermore, the immunochromatographic test reader according to the present invention has an illuminating optical system that illuminates the measuring light onto the immunochromatographic test strip and a detecting optical system that detects the reflected light from the immunochromatographic test strip resulting from the illumination of the measuring light by the illuminating optical system. Here, the holding section of the cartridge preferably includes a recess of a shape corresponding to the outer shape of the immunochromatographic test utensil and this recess preferably has a depth such that the optical axis of the illuminating optical system and the optical axis of the detecting optical system intersect at a surface of the immunochromatographic test strip included in the immunochromatographic test utensil in the state of being fitted into the recess. The immunochromatographic test reader according to the present invention further comprises a structure for exposing the through hole, provided in the cartridge, to the exterior of the reader in the state wherein the cartridge is introduced in the reader.

In the immunochromatographic test reader according to the present invention, the illuminating optical system and the detecting optical system are preferably held by an optical head as a pair of optical systems. In this case, the reader has a scanning mechanism that moves the cartridge introduced in the reader and the optical head in a relative manner.

It is preferable that the scanning mechanism moves the cartridge and the optical head relative to each other at a first speed during a period, in which the measuring light is illuminated onto the immunochromatographic test strip via a window provided in the immunochromatographic test utensil, and moves the cartridge and the optical head relative to each other at a second speed higher than the first speed during a period, in which the measuring light is illuminated onto portions besides the window provided in the immunochromatographic test utensil. This is because the scanning time can then be shortened without lowering the measurement precision.

An immunochromatographic test strip inspection system according to the present invention has the immunochromatographic test reader with the above-described structure and a means that stores, in associated form, information concerning the immunochromatographic test utensil, which are stored in the information storage means provided in the cartridge, and information concerning colored regions read by the reader.

In the immunochromatographic test strip inspection system according to the present invention with the above-described arrangement, the information concerning the immunochromatographic test utensil, which are stored in the information storage means, and the information concerning the colored regions read by the reader are stored in associated form. The information concerning the immunochromatographic test utensil, which are stored in the information storage means, and the information concerning the colored regions read by the reader can thus be managed accurately in associated form.

The information concerning the colored regions read by the reader are preferably sent to the cartridge and stored in the information storage means of the cartridge. In this case, the immunochromatographic test utensil held in the cartridge, the information concerning the immunochromatographic test utensil, which are stored in the information storage means, and the information concerning the colored regions read by the reader can be managed accurately in associated form.

The present invention will be more fully understood from the detailed description given hereinbelow and the accompanying drawings, which are given by way of illustration only and are not to be considered as limiting the present invention.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will be apparent to those skilled in the art from this detailed description.

### Effect of the Invention

In accordance with the present invention, an immunochromatographic test reader, a cartridge applicable to the reader, and an immunochromatographic test strip inspection system, which enable accommodation of a plurality of casings that differ in outer shape and enable immunochromatographic test utensils to be managed readily in association with information concerning the immunochromatographic test utensils, can be provided.

### Brief Description of the Drawings

Fig. 1 shows a plan view and a perspective view of an example of an immunochromatographic test utensil;
Fig. 2 shows a plan view and a perspective view of an example of an immunochromatographic test utensil;
Fig. 3 shows a plan view, a perspective view, and a front view of an arrangement of an example of a cartridge according to the present invention;
Fig. 4 shows a plan view, a perspective view, and a front view of an arrangement of an example of a cartridge according to the present invention;
Fig. 5 shows perspective views of an example of an immunochromatographic test utensil and a cartridge in a state wherein the immunochromatographic test utensil is held in the cartridge and a state wherein the immunochromatographic test utensil is removed from the cartridge;
Fig. 6 is a block diagram for explaining the arrangement of the cartridge shown in Fig. 3;
Fig. 7 is a block diagram for explaining the arrangement of the cartridge shown in Fig. 4;
Fig. 8 is a perspective view of an arrangement of a reader according to the present invention;
Fig. 9 is a perspective view of the arrangement of the reader according to the present invention;
Fig. 10 is a schematic plan view of a cover body included in the reader as viewed from a rear surface side;
Fig. 11 is a schematic plan view of the reader in a state wherein the cover body has been removed;
Fig. 12 is a block diagram of an arrangement of the reader according to the present invention;
Fig. 13 is a perspective view of a state wherein the cartridge is being removed from the reader shown in Fig. 9;
Fig. 14 is a plan view of a state wherein the cartridge is introduced into a chassis;
Fig. 15 is a schematic section view of an optical head of the reader according to the present invention;
Fig. 16 is a flowchart for explaining operations of the reader according to the present invention;
Fig. 17 shows a plan view and a side view of an example of an immunochromatographic test utensil;
Fig. 18 is an absorbance profile of reflected light of an immunochromatographic test strip;
Fig. 19 is a block diagram for explaining an example of an arrangement of an immunochromatographic test strip inspection system according to the present invention; and
Fig. 20 is a block diagram for explaining an example of an arrangement of an immunochromatographic test strip inspection system according to the present invention.

### Description of the Reference Numerals

1...reader; 21...controlling circuit section; 21a...CPU; 21b...memory section; 31...measuring section; 35... optical head; 37...scanning mechanism; 39...connecting terminal section; 41...illumination optical system; 43a, 43b...light emitting element; 51...detecting optical system; 53...light receiving element; 63...driving motor; AW1, AW2... sample attachment window; C1, C2...casing; CA1, CA2...cartridge; CL...colored line; CT1, CT2...connecting terminal section; H1, H2...recess; IM1, IM2...immunochromatographic test strip; IM1b, IM2b...detecting section; IS1, IS2...information storage section; OW1, OW2...sample observation window; P1...partition; PC... external information processing apparatus; S1-S4...index; and TE1, TE2...immunochromatographic test utensil.

### Best Modes for Carrying Out the Invention

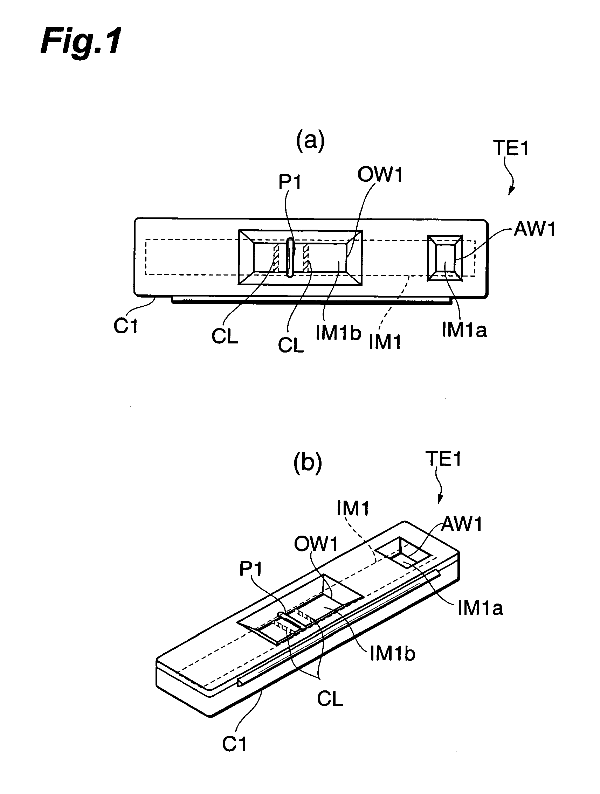

Embodiments of immunochromatographic test reader, cartridge applicable to the reader, and immunochromatographic test strip inspection system according to the present invention shall now be described in detail using Figs. 1-20. In the description of the drawings, the same portions or the same elements are provided with the same symbols and redundant description shall be omitted.

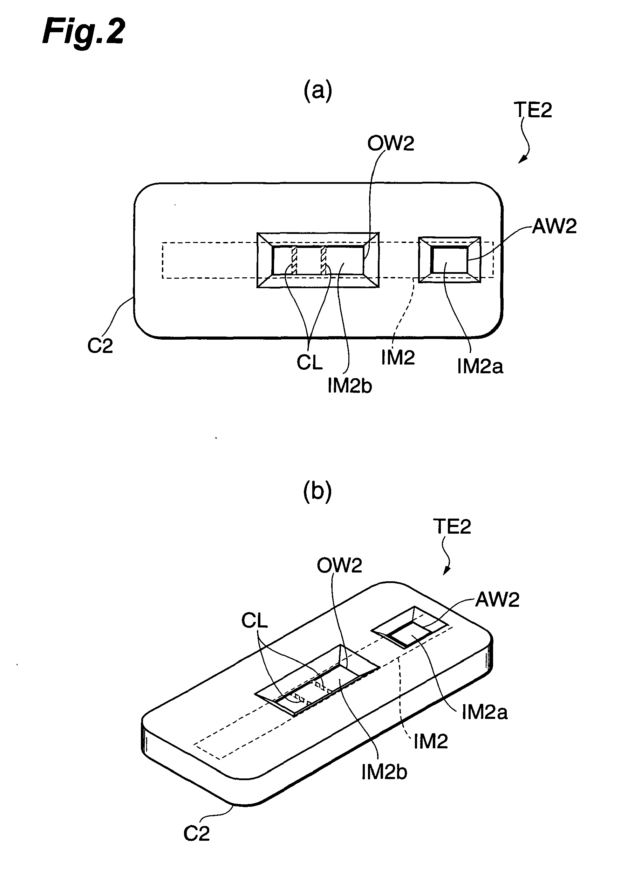

Arrangements of immunochromatographic test utensils TE1 and TE2 shall first be explained with reference to Figs. 1 and 2. The area (a) shown in Fig. 1 is a plan view of immunochromatographic test utensil TE1 and the area (b) shown in Fig. 1 is a perspective view of immunochromatographic test utensil TE1. The area (a) shown in Fig. 2 is a plan view of immunochromatographic test utensil TE2 and the area (b) shown in Fig. 2 is a perspective view of immunochromatographic test utensil TE2.

As shown the areas (a) and (b) of Fig. 1, the immunochromatographic test utensil TE1 has a casing C1 with a rectangular shape in planar view and an immunochromatographic test strip IM1 that is held in the casing C1. The size of casing C1 is 15mm width × 70mm depth × 5mm thickness. The immunochromatographic test utensil TE1 is, for example, an immunochromatographic test utensil for A-type influenza virus inspection.

The casing C1 has, along its longitudinal direction, a plurality of windows that are openings that expose the immunochromatographic test strip IM1 to the exterior of casing C1, that is, a sample attachment window AW1 for dropping of a sample and an observation window OW1 that exposes colored portions of the immunochromatographic test strip IM1 to the exterior. The edge portions that form the sample attachment window AW1 and the edge portions that form the observation window OW1 are tapered so as to incline towards the immunochromatographic test strip IM1. In the immunochromatographic test utensil TE1, a portion of the observation window OW1 is partitioned by a partition P1 and used as a control window.

As shown in the areas (a) and (b) of Fig. 2, the immunochromatographic test utensil TE2 has a casing C2 with a rectangular shape in planar view and an immunochromatographic test strip IM2 that is held in the casing C2. The size of casing C2 is 30mm width × 70mm depth × 5mm thickness. The immunochromatographic test utensil TE2 is, for example, an immunochromatographic test utensil for O-157 inspection.

The casing C2 has, along its longitudinal direction, a plurality of windows that are openings that expose the immunochromatographic test strip IM2 to the exterior of casing C2, that is, a sample attachment window AW2 for dropping of a sample and an observation window OW2 that exposes colored portions of the immunochromatographic test strip IM2 to the exterior. The edge portions that form sample attachment window AW2 and the edge portions that form observation window OW2 are tapered so as to incline towards the immunochromatographic test strip IM2.

The immunochromatographic test strips IM1 and IM2 are formed of a nitrocellulose membrane, filter paper, or other material and has a rectangular shape. The immunochromatographic test strips IM1 and IM2 respectively have sample attachment portions IM1a and IM2a, provided at positions corresponding to the sample attachment windows AW1 and AW2, and detection portions IM1b and IM2b, provided at positions corresponding to the observation windows OW1 and OW2. The detection portions IM1b and IM2b have antibodies (or antigens) applied and fixed thereto which respectively react with antigens (or antibodies) in a sample and have line-like (or band-like or point-like) forms.

A sample is dropped onto the sample attachment portion IM1a or IM2a of the immunochromatographic test strip IM1 or IM2 from the sample attachment window AW1 or AW2. Antigens (or antibodies) in the sample bind with label dyes, and bound combinations of the antigens (or antibodies) in the sample and the label dyes and unreacted label dyes move in the longitudinal direction of the immunochromatographic test strip IM1 or IM2. Here, suppose that antigens are contained in the sample and the antigens undergo antigen-antibody reactions with the detection portion IM1b or IM2b. In accompaniment with the movement of the sample, the antigens in the sample react specifically with antibodies fixed to the detection portion IM1b or IM2b and line-like patterns (colored lines CL), colored by the label dyes, form at the detection portion IM1b or IM2b that has reacted. These colored lines CL form so as to extend in a direction that intersects (for example, the direction orthogonal to) the direction of movement of the antigens (or antibodies) in the sample in the immunochromatographic test strip IM1 or IM2 and can be observed from the observation window OW1 or OW2. The width of a colored line CL is, for example, approximately 1.0mm. The length of a colored line CL in the longitudinal direction is, for example, approximately 5mm.

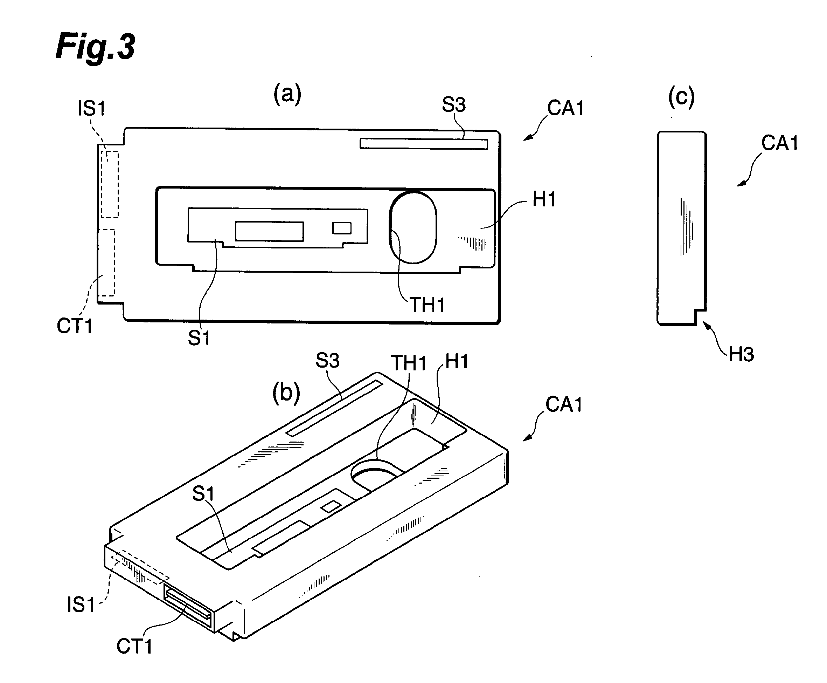

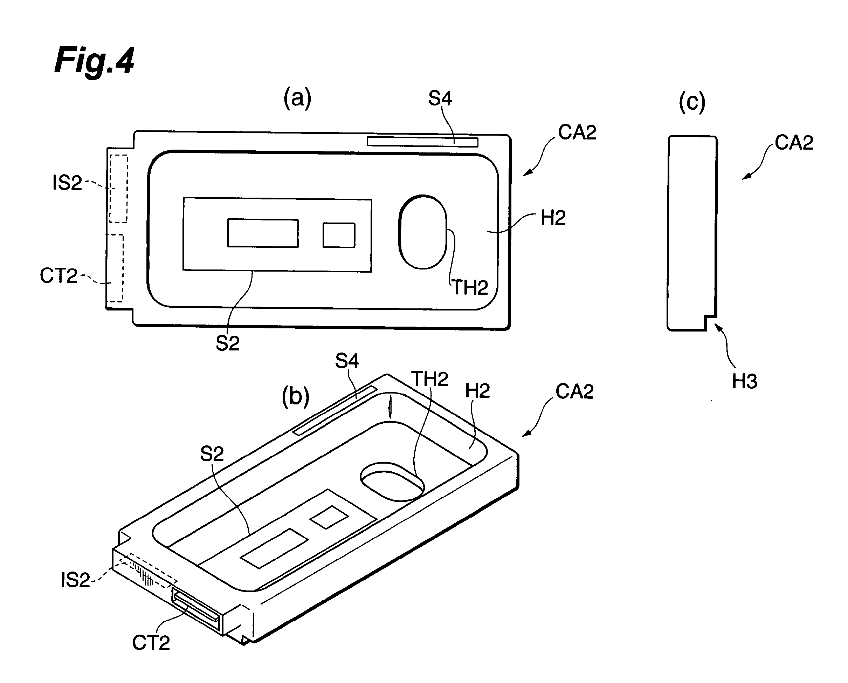

The arrangements of cartridges CA1 and CA2 shall now be explained with reference to Figs. 3 and 4. The area (a) shown in Fig. 3 is a plan view of the cartridge CA1, the area (b) shown in Fig. 3 is a perspective view of the cartridge CA1, and the area (c) shown in Fig. 3 is a front view of the cartridge CA1. The area (a) shown in Fig. 4 is a plan view of the cartridge CA2, the area (b) shown in Fig. 4 is a perspective view of the cartridge CA2, and the area (c) shown in Fig. 4 is a front view of the cartridge CA2.

The cartridge CA1 and the cartridge CA2 have the same outer shape and have a substantially rectangular shape in plan view. Each of the cartridge CA1 and the cartridge CA2 has a size of 35mm width × 75mm depth × 8mm thickness.

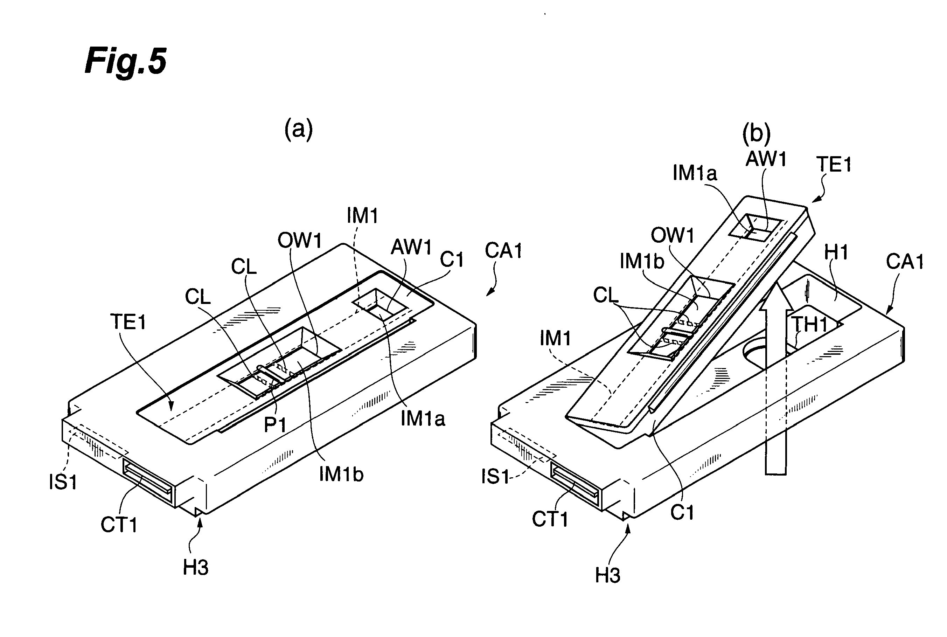

The cartridge CA1 is a member that corresponds to the immunochromatographic test utensil TE1 and, as shown in the areas (a) and (b) of Fig. 3, is provided with a recess H1. The recess H1 is formed to correspond to the outer shape of the casing C1, and the casing C1 is fitted into the recess H1 (see the area (a) shown Fig. 5). By the casing C1 being fitted into the recess H1, the immunochromatographic test utensil TE1 is held by the cartridge CA1. The recess H1 functions as a holding section that holds the immunochromatographic test utensil TE1.

The cartridge CA1 is provided with a through hole TH1 that communicates with the recess H1. The through hole TH1 is formed at a position that overlaps with the casing C1, which has been fitted into and is held by the recess H1, and is formed to pass through from the surface at the side at which the casing C1 is held (fitted) to the surface at the opposite side. By inserting a finger or a tool, etc., into through hole TH1 and pressing the casing C1 in the state in which the casing C1 is fitted into the recess H1, the immunochromatographic test utensil TE1 is pushed out of the cartridge CA1 as shown in the area (b) of Fig. 5. Since the immunochromatographic test utensil TE1 can thus be taken out readily from the cartridge CA1, the possibility of directly touching a connecting terminal section CT1 of the cartridge CA1 to be described later, the sample attachment window AW1, the observation window OW1, the portions peripheral to the above portions, or the immunochromatographic test strip IM1 through any of the windows is extremely low, thus providing excellent protection of the connecting terminal section as well as excellent sanitation.

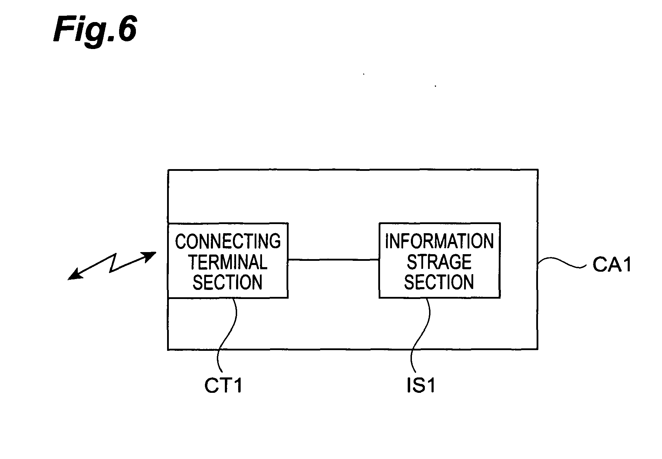

In order to enable storage of information and communication of the information with the exterior, the cartridge CA1 has an information storage section IS1 and a connecting terminal section CT1, connected to information storage section IS1, as shown in Fig. 6. The information storage section IS1 stores information concerning the immunochromatographic test utensil TE1 held by the cartridge CA1. When connected to a connecting terminal section 39 of a reader 1 to be described later, the connecting terminal section CT1 enables sending of the information concerning the immunochromatographic test utensil TE1 from the information storage section IS1. Fig. 6 is a block diagram for explaining the arrangement of the cartridge CA1.

The cartridge CA2 is a member that corresponds to the immunochromatographic test utensil TE2 and, as shown in the areas (a) and (b) of Fig. 4, is provided with a recess H2. The recess H2 is formed to correspond to the outer shape of the casing C2, and the casing C2 is fitted into the recess H2. By the casing C2 being fitted into the recess H2, the immunochromatographic test utensil TE2 is held by the cartridge CA2. The recess H2 functions as a holding section that holds the immunochromatographic test utensil TE2.

The cartridge CA2 is provided with a through hole TH2 that communicates with the recess H2. The through hole TH2 is formed at a position that overlaps with the casing C2, which has been fitted into the recess H2, and is formed from the surface at the side at which the casing C2 is held (fitted) to the surface at the opposite side. By inserting a finger or a tool, etc., into the through hole TH2 and pressing the casing C2 in the state in which the casing C2 is fitted into the recess H2, the immunochromatographic test utensil TE2 is pushed out of the cartridge CA2 in the same manner as the immunochromatographic test utensil TE1. Since the immunochromatographic test utensil TE2 can thus be taken out readily from the cartridge CA2, the possibility of directly touching a connecting terminal section CT2 of the cartridge CA2 to be described later, the sample attachment window AW2, the observation window OW2, the portions peripheral to the above portions, or the immunochromatographic test strip IM2 through any of the windows is extremely low, thus providing excellent protection of the connecting terminal section as well as excellent sanitation.

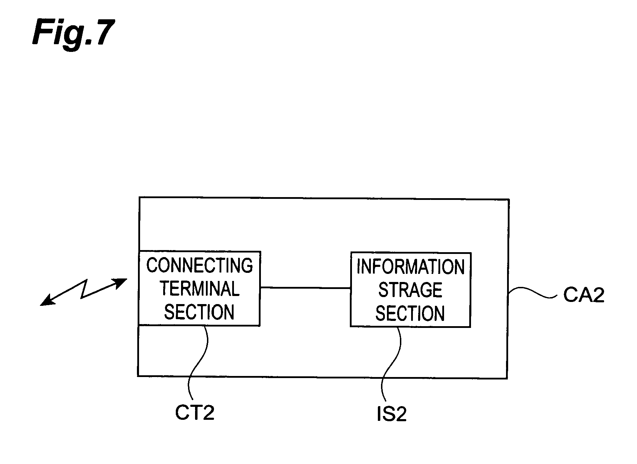

In order to enable storage of information and communication of the information with the exterior, the cartridge CA2 has an information storage section IS2 and the connecting terminal section CT2, connected to the information storage section IS2, as shown in Fig. 7. The information storage section IS2 stores the information concerning the immunochromatographic test utensil TE2 held by the cartridge CA2. When connected to the connecting terminal section 39 of the reader 1 to be described later, the connecting terminal section CT2 enables sending of the information concerning the immunochromatographic test utensil TE2 from the information storage section IS2. Fig. 7 is a block diagram for explaining the arrangement of the cartridge CA2.

For example, IC chips with a ROM, RAM, or EEPROM, barcodes, magnetic stripes, etc., may be used as information storage sections IS1 and IS2. Metal terminals may be used as connecting terminal section CT1 and CT2. In place of the connecting terminal sections CT1 and CT2, the information transmitting means that can send and receive information in a non-contacting manner may be provided. As such information transmitting means, means suited to the devices (the above-mentioned IC chip, barcode, magnetic strip, etc.) that make up information storage sections IS1 and IS2 are selected.

The information concerning the immunochromatographic test utensil TE1 or TE2 include the information concerning an inspection performed using the immunochromatographic test utensil TE1 or TE2, the information that identify the immunochromatographic test utensil TE1 or TE2, the information concerning a colored region reading process corresponding to the immunochromatographic test strip IM1 or IM2 included in the immunochromatographic test utensil TE1 or TE2, the information concerning a colored region reading process corresponding to the casing C1 or C2 included in the immunochromatographic test utensil TE1 or TE2, the information concerning a method of reading by the reader 1, the information concerning methods of performing computations on data obtained by the reader 1, the information concerning colored regions read by the reader 1, etc.

In the case where the information concerning the immunochromatographic test utensil TE1 or TE2 include the information concerning the inspection performed using the immunochromatographic test utensil TE1 or TE2, the immunochromatographic test utensil TE1 or TE2 and the information concerning the inspection using the immunochromatographic test utensil TE1 or TE2 can be managed readily and accurately in associated form. The information concerning the inspection using the immunochromatographic test utensil TE1 or TE2 include the name of an inspection subject, the date of inspection, the name of the person in charge of inspection, etc.

In the case where the information concerning the immunochromatographic test utensil TE1 or TE2 include the information that identify the immunochromatographic test utensil TE1 or TE2, the immunochromatographic test utensil TE1 or TE2 and the information that identify the immunochromatographic test utensil TE1 or TE2 can be managed readily and accurately in associated form. The information that identify the immunochromatographic test utensil TE1 or TE2 include the name of a manufacturer, the manufactured serial number, an item number, the name of an inspection item (for example, Salmonella inspection, endocrine disrupter inspection, influenza inspection, medical drug inspection, etc.), etc., of each immunochromatographic test utensil TE1 or TE2.

In the case where the information concerning the immunochromatographic test utensil TE1 or TE2 include the information concerning the colored region reading process corresponding to the immunochromatographic test strip IM1 or IM2 included in the immunochromatographic test utensil TE1 or TE2, the immunochromatographic test utensil TE1 or TE2 and the information concerning the colored region reading process corresponding to the immunochromatographic test strip IM1 or IM2 included in the immunochromatographic test utensil TE1 or TE2 can be managed readily and accurately in associated form. Here, the information concerning the colored region reading process corresponding to the immunochromatographic test strip IM1 or IM2 included in the immunochromatographic test utensil TE1 or TE2 include a waiting time until the start of scanning (reading), the number of scans, the number of colored lines CL, the positions of colored lines, CL etc.

In the case where the information concerning the immunochromatographic test utensil TE1 or TE2 include the information concerning the colored region reading process corresponding to the casing C1 or C2 included in the immunochromatographic test utensil TE1 or TE2, the immunochromatographic test utensil TE1 or TE2 and the information concerning the colored region reading process corresponding to the casing C1 or C2 included in the immunochromatographic test utensil TE1 or TE2 can be managed readily and accurately in associated form. Here, the information concerning the colored region reading process corresponding to the casing C1 or C2 included in the immunochromatographic test utensil TE1 or TE2 include the position of the observation window OW1 or OW2 for exposing the immunochromatographic test strip IM1 or IM2, the size of the observation window OW1 or OW2, the existence of the partition P1 in the observation window OW1 or OW2, etc.

In the case where the information concerning the immunochromatographic test utensil TE1 or TE2 include the information concerning the method of reading by the reader 1, the immunochromatographic test utensil TE1 or TE2 and the information concerning the method of reading by the reader 1 can be managed readily and accurately in associated form. The information concerning the method of reading by the reader 1 include the measurement precision, the scanning speed, the number of times of data accumulation, etc.

In the case where the information concerning the immunochromatographic test utensil TE1 or TE2 include the information concerning the methods of performing computations on the data obtained by the reader 1, the immunochromatographic test utensil TE1 or TE2 and the information concerning the methods of performing computations on the data obtained by the reader 1 can be managed readily and accurately in associated form. The information concerning the methods of performing computations on the data obtained by the reader 1 include methods of performing computations on the data obtained by reading of colored regions (computation of average value, computation of peak value, computation of absorbance, etc.), methods of judging the data obtained by reading of the colored regions, scattering correction of coloration characteristics according to lot, etc.

In the case where the information concerning the immunochromatographic test utensil TE1 or TE2 include the information concerning the colored regions read by the reader 1, the immunochromatographic test utensil TE1 and TE2 and the information concerning the colored regions read by the reader 1 can be managed readily and accurately in associated form. The information concerning the colored regions read by the reader 1 include data obtained by reading of the colored regions, judgment results based on the data, etc.

On the surface that defines the bottom of the recess H1 or H2 in the cartridge CA1 or CA2 is indicated a label (in the present embodiment, a drawing of the corresponding the immunochromatographic test utensil TE1 or TE2) S1 or S2 for identifying the correction direction in which the corresponding the immunochromatographic test utensil TE1 or TE2 is fitted. Also indicated on the cartridge CA1 or CA2 is a label (in the present embodiment, characters) S3 or S4 that indicates the information concerning the corresponding the immunochromatographic test utensil TE1 or TE2 (for example, the above-mentioned information that identify the immunochromatographic test utensil TE1 or TE2).

On one of the side portions parallel to the long side of each of the cartridges CA1 and CA2 is formed a recess H3 that defines the direction of insertion of the corresponding the cartridge CA1 or CA2 into the reader 1 to be described later.

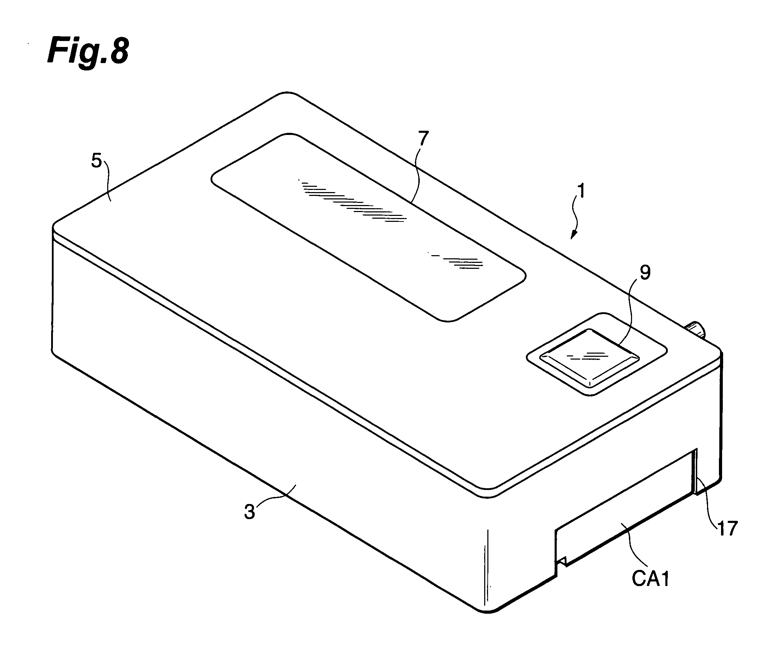

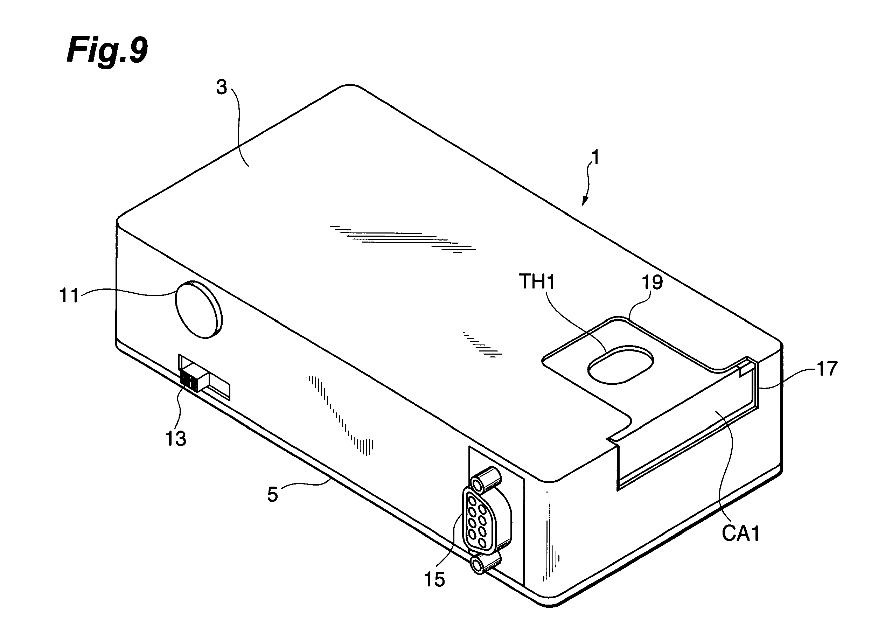

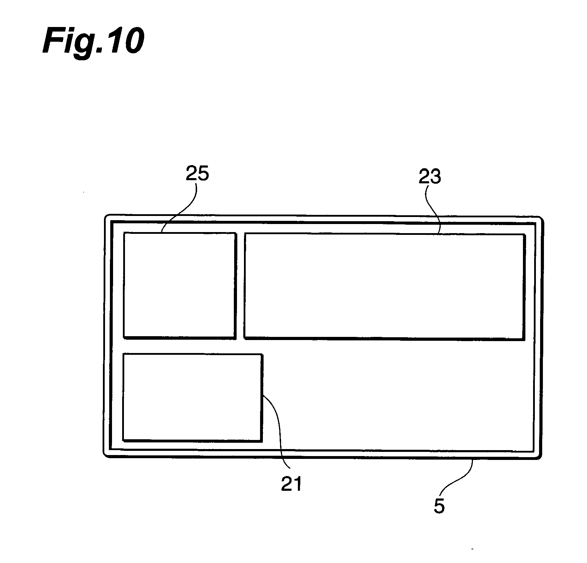

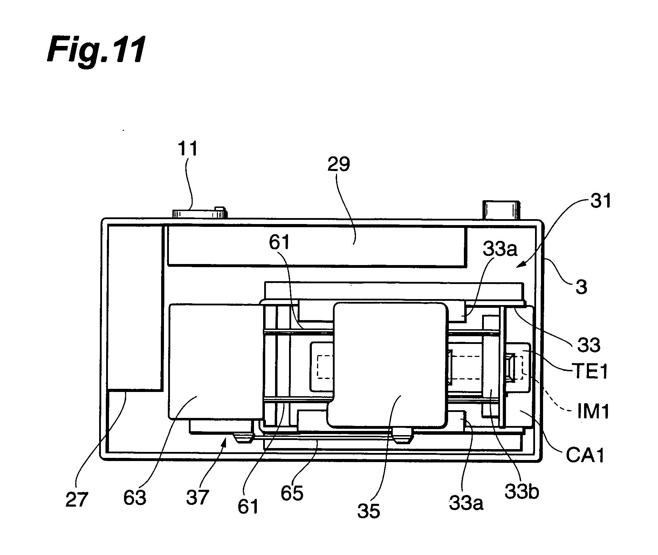

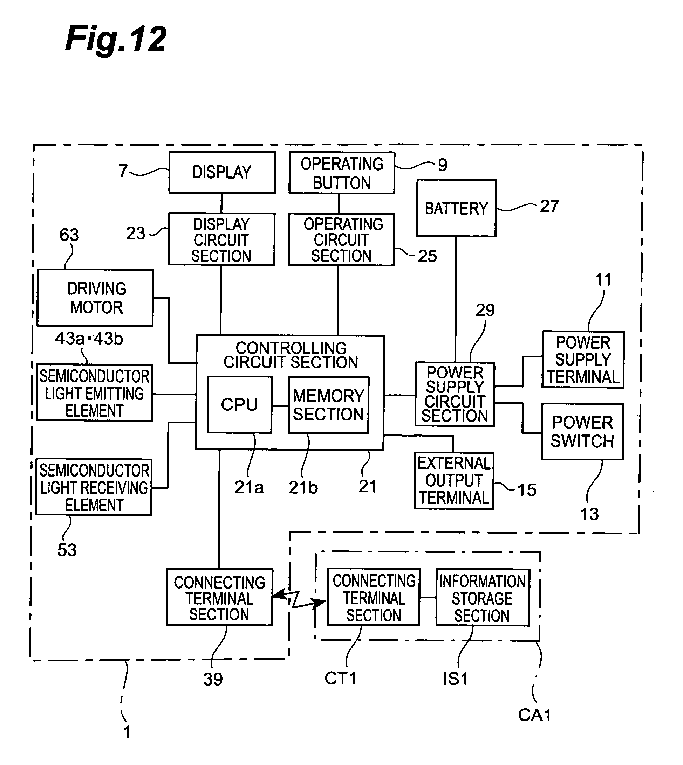

An arrangement of the reader 1 shall now be described with reference to Figs. 8-12. Figs. 8 and 9 are perspective views of the reader 1. Fig. 10 is a schematic plan view of a cover body 5, included in the reader 1, as viewed from a rear surface side, and Fig. 11 is a schematic plan view of the reader 1 in a state wherein the cover body 5 is removed. Fig. 12 is a block diagram for explaining the arrangement of the reader 1. Figs. 8-18 illustrate a case where the colored lines CL, formed on the immunochromatographic test utensil TE1, are to be read by the reader 1.

The reader 1 illuminates measuring light onto the colored lines CL formed on the immunochromatographic test strip IM1 or IM2 and measures the coloration of the colored lines CL by receiving the reflected light. As shown in Fig. 8, the reader 1 has a housing 3, with a box-like shape and opened on the upper face, and a cover body 5, which closes the opening of housing 3. A display 7, which displays measurement results, etc., and an operation button 9 for starting measurement operations, are disposed on the cover body 5.

As shown in Fig. 9, on a side face of the housing 3 are disposed a power supply terminal 11, which is connected to a power supply line from an external power supply, a power supply switch 13, and an external output terminal 15 for outputting the measurement results and other data to an external information processing apparatus. Also, on a side face of the housing 3 is formed an insertion slot 17 for introducing the cartridge CA1 or CA2 into the reader 1.

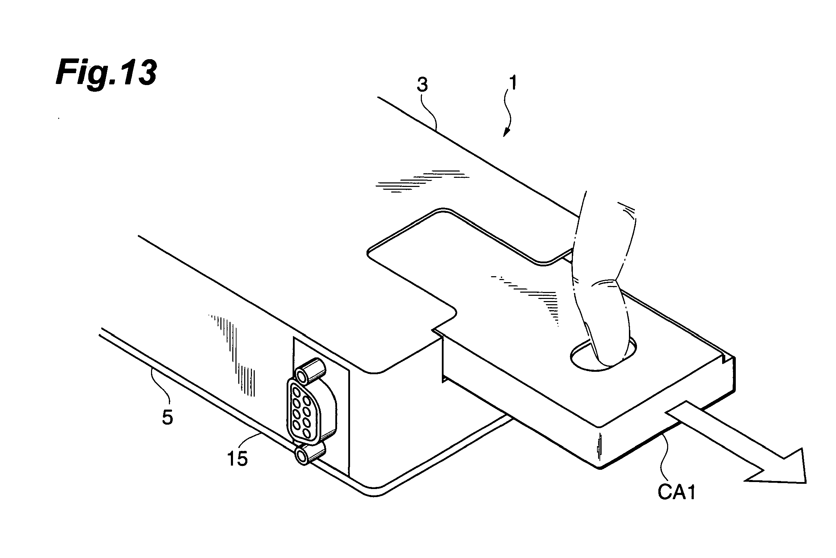

A slit 19 that is continuous with the insertion slot 17 is provided in the bottom surface of the housing 3. As shown in Fig. 9, this slit 19 is formed so that the through hole TH1 or TH2, formed in the cartridge CA1 or CA2, is exposed from the reader 1 in the state where the cartridge CA1 or CA2 is introduced into the reader 1 from the insertion slot 17. As shown in Fig. 13, the cartridge CA1 or CA2 is taken out from the reader 1 by inserting a finger or a tool, etc., into the through hole TH1 or TH2 through the slit 19 in the state where the cartridge CA1 or CA2 is introduced and thereby drawing out the cartridge CA1 or CA2.

As shown in Fig. 12, the reader 1 has a controlling circuit section 21 for controlling the operations, etc., of the reader 1, a displaying circuit section 23 for controlling the display on display 7, and an operating circuit section 25 for outputting a signal corresponding to the operation state of the operation button 9. As shown in Fig. 10, the controlling circuit section 21, the displaying circuit section 23, and the operating circuit section 25 are disposed on the rear surface of housing 5. Reader 1 also has a battery 27, a power supply circuit section 29, and a measuring section 31. As shown in Fig. 11, the battery 27, the power supply circuit section 29, and the measuring section 31 are disposed inside the housing 3. The controlling circuit section 21 includes a CPU 21a and a ROM or other memory section 21b.

In order to illuminate measuring light onto the immunochromatographic test strip IM1 or IM2, included in the immunochromatographic test utensil TE1 or TE2 held in the introduced cartridge CA1 or CA2, and detect the reflected light from the immunochromatographic test utensil TE1 or TE2, the measuring section 31 is arranged at a position corresponding to the insertion slot 17. This measuring section 31 includes a chassis 33, an optical head 35, a scanning mechanism 37, etc.

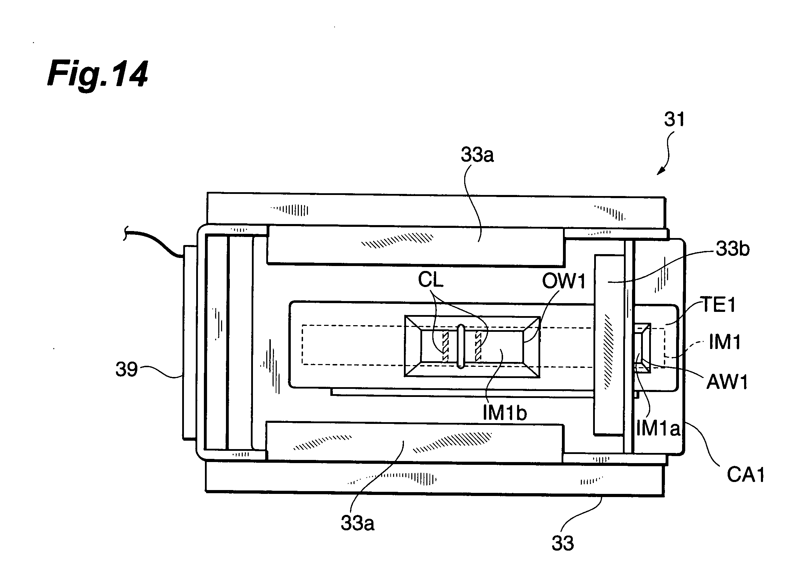

The chassis 33 is arranged in correspondence to the insertion slot 17 and is fixed to the housing 3. The cartridge CA1 or CA2, which is inserted from the insertion slot 17 is introduced inside the chassis 33 as shown in Fig. 14. Fig. 14 shows a state where the cartridge CA1, holding the immunochromatographic test utensil TE1, is introduced into the chassis 33. In Fig. 14, the optical head 35 and the scanning mechanism 37 are omitted.

The chassis 33 has protrusions 33a that are positioned above the respective sides of the introduced cartridge CA1 or CA2 and prevent the optical head 35 and the cartridge CA1 from contacting each other. The protrusions 33a extend in the direction of introduction of the cartridge CA1 or CA2. The chassis 33 also has a stopper 33b that is positioned above the immunochromatographic test utensil TE1 or TE2 held in the introduced cartridge CA1 or CA2. This stopper 33b contacts the immunochromatographic test utensil TE1 or TE2 and prevents the casing C1 or C2 (immunochromatographic test utensil TE1 or TE2) from being pressed erroneously and thereby prevents the immunochromatographic test utensil TE1 or TE2 from being pushed out and removed from the cartridge CA1 or CA2 when, for example, a finger is inserted into the through hole TH1 or TH2 to take out the cartridge CA1 or CA2 from the reader 1.

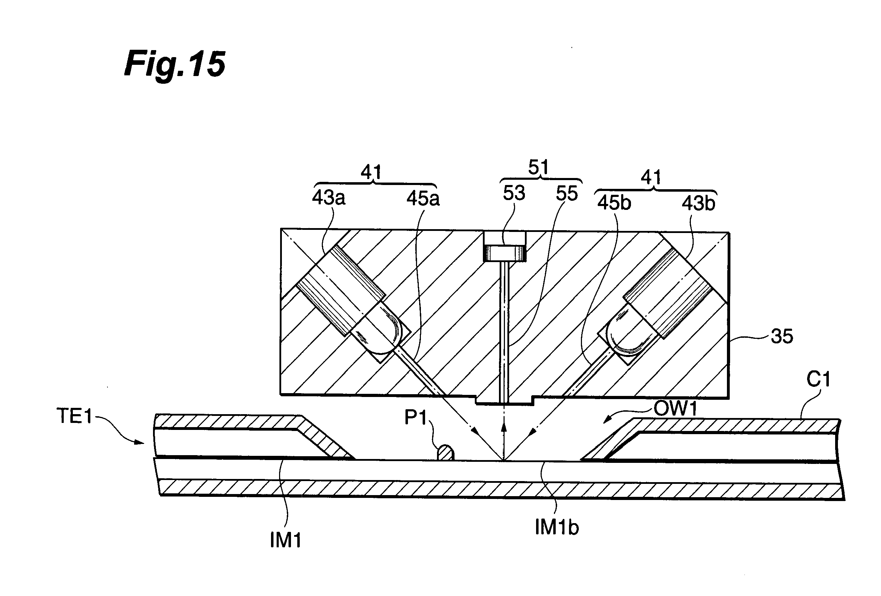

As shown in Fig. 15, the optical head 35 is provided with an illuminating optical system 41, which illuminates measuring light onto the immunochromatographic test strip IM1 or IM2 in the immunochromatographic test utensil TE1 or TE2, held in the introduced cartridge CA1 or CA2, and a detecting optical system 51, which detects reflected light from the immunochromatographic test strip IM1 or IM2.

The illuminating optical system 41 has a pair of light emitting elements 43a and 43b and holes 45a and 45b, provided in correspondence to the light emitting elements 43a and 43b. In the present embodiment, the light emitting diodes (LEDs), which are semiconductor light emitting elements, are employed as the light emitting elements 43a and 43b.

The light emitting elements 43a and 43b are arranged in parallel in a scanning direction (direction substantially orthogonal to the colored lines CL formed on the immunochromatographic test strip IM1 or IM2) of the optical head 35 and mounted to the optical head 35. The light, emitted from the light emitting elements 43a and 43b, passes through the holes 45a and 45b, and outputs from the optical head 35 towards the immunochromatographic test strip IM1 or IM2.

The hole 45a has the light emitting element 43a positioned at one end thereof and is formed to extend downward while inclining forward along the scanning direction of the optical head 35 from the one end. The hole 45b has the light emitting element 43b positioned at one end thereof and is formed to extend downward while inclining rearward along the scanning direction of the optical head 35 from the one end. Each of the holes 45a and 45b functions as a slit and the inner diameter thereof is set, for example, to approximately 1mm.

As shown in Fig. 15, the detecting optical system 51 has a light receiving element 53 and a hole 55, provided in correspondence to the light receiving element 53. With the present embodiment, a silicon (Si) photodiode, which is a semiconductor light receiving element, is employed as the light receiving element 53.

The light receiving element 53 is arranged at an intermediate position between the light emitting element 43a and the light emitting element 43b as viewed in the scanning direction of the optical head 35, and is mounted to the optical head 35. The reflected light from the immunochromatographic test strip IM1 or IM2 passes through the hole 55, and is made incident on the light receiving element 53.

The hole 55 has the light receiving element 53 positioned at one end thereof and is formed to extend in a direction substantially orthogonal to the immunochromatographic test strip IM1 or IM2 from the one end. Each of the hole 55 functions as a slit and the inner diameter thereof is set, for example, to approximately 0.5mm.

Here, the depth of the recess H1 or H2 of the cartridge CA1 or CA2 is set so that the optical axes of the illuminating optical system 41 and the optical axis of the detecting optical system 51 intersect at the top surface of the immunochromatographic test strip IM1 or IM2 held by the casing C1 or C2 in the state of being fitted in the recess H1 or H2. Thus regardless of the type of the immunochromatographic test utensil TE1 or TE2, the optical axis of the light emitting element 43a (illuminating optical system 41), which is positioned to the rear as viewed in the scanning direction of the optical head 35, the optical axis of the light emitting element 43b (illuminating optical system 41), which is positioned to the front as viewed in the scanning direction of the optical head 35, and the optical axis of the light receiving element 53 (detecting optical system 51) intersect with each other at the top surface of the immunochromatographic test strip IM1 or IM2 as shown in Fig. 15.

In the present embodiment, the pair of the light emitting elements 43a and 43b are positioned so as to be bilaterally symmetrical in the longitudinal direction of the cartridge CA1 or CA2, with the optical axis of the light receiving element 53 as the central axis. Thus even if a colored line CL exists near an edge of the observation window OW1 or OW2 in the longitudinal direction of the cartridge CA1 or CA2, that is to be more detailed, even if a colored line CL exists on a portion of the immunochromatographic test strip IM1 or IM2 that is exposed from the observation window OW1 or OW2 and is located near the boundary of the casing C1 or C2 in the longitudinal direction of the cartridge CA1 or CA2, the light emitted from at least one of the light emitting elements 43a and 43b will definitely be illuminated onto the colored line CL. Since the region for detecting the colored lines CL is thus broadened, more diverse types of casings can be accommodated.

As shown in Fig. 11, the scanning mechanism 37 has a pair of the guide rails 61 that slidably guides the optical head 35 in the scanning direction that perpendicularly crosses the colored lines CL formed on the immunochromatographic test strip IM1 or IM2, a driving motor 63, and a crank mechanism section 65 that transmits the driving force of the driving motor 63 to the optical head 35. The scanning mechanism 37 moves the optical head 35 reciprocatingly along the scanning direction. The guide rails 61 are fixed to the chassis 33 and extend in the introduced direction of the cartridge CA1 or CA2. The driving motor 63 is also fixed to the chassis 33.

In the scanning mechanism 37, the rotating motion of the driving motor 63 is converted by the crank mechanism section 65 into a reciprocating motion and transmitted to the optical head 35, and the optical head 35 is thereby moved in the scanning direction while being guided by the pair of left and right guide rails 61. As a result, the optical head 35 moves with respect to the chassis 33 in the scanning direction that perpendicularly crosses the colored lines CL formed on the immunochromatographic test strip IM1 or IM2. In the present embodiment, when the driving motor 63 completes a single turn, the optical head 35 completes a single reciprocation. The mechanism for transmitting the driving force of the driving motor 63 to the optical head 35 is not restricted to the above crank mechanism section 65, and a rack and pinion mechanism or other power transmitting mechanism may be applied instead.

The driving motor 63 of the scanning mechanism 37 is connected via a driving circuit section (not shown) to the controlling circuit section 21 and performs driving based on a signal output from the controlling circuit section 21. The controlling circuit section 21 performs a rotation control of the driving motor 63 of the scanning mechanism 37.

The light emitting elements 43a and 43b emit light based on signals output from the controlling circuit section 21. While the optical head 35 is moved in the scanning direction by the rotation of the driving motor 63, the controlling circuit section 21 makes the light emitting elements 43a and 43b become lit and makes measuring light (slit light) be illuminated onto the detection portion IM1b or IM2b of the immunochromatographic test strip IM1 or IM2 that is exposed via the observation window OW1 or OW2 of the casing C1 or C2.

The output of the light receiving element 53 is connected to the controlling circuit section 21. The controlling circuit section 21 inputs the detection signal from the light receiving element 53 that has received the reflected light, from the detection portion IM1b or IM2b of the immunochromatographic test strip IM1 or IM2, resulting from the lighting up of the light emitting elements 43a and 43b.

The connecting terminal section 39, to which the connecting terminal section CT1 or CT2 can be connected, is provided on the chassis 33. The connecting terminal section 39 is connected to the controlling circuit section 21, and the connecting terminal section 39 is a circuit part, which, in the state of being connected to the connecting terminal section CT1 or CT2, sends the information concerning the immunochromatographic test utensil TE1 that are stored in the information storage section IS1 or IS2 to the controlling circuit section 21. If, for example, information transmitting means enabling the sending and receiving of information in a non-contacting manner is employed in place of the connecting terminal section CT1 or CT2, information transmitting means corresponding to the above information transmitting means is employed in place of the connecting terminal section 39.

Though in the above-described embodiment, the scanning mechanism 37 moves the optical head 35 with respect to the cartridge CA1 or CA2, the cartridge CA1 or CA2 may be moved with respect to the optical head 35 or both the optical head 35 and the cartridge CA1 or CA2 may be moved instead. That is, the scanning mechanism 37 moves the optical head 35 and the cartridge CA1 or CA2 relative to each other. Here, it is preferable for the cartridge CA1 or CA2 and the optical head 35 to be moved relative to each other at a first speed during a period in which measuring light is illuminated onto the immunochromatographic test strip IM1 or IM2 via the window OW1 or OW2 provided in the casing C1 or C2 and for the cartridge CA1 or CA2 and the optical head 35 to be moved relative to each other at a second speed higher than the first speed during a period in which measuring light is illuminated at a portion of the casing C1 or C2 other than the window OW1 or OW2. The scanning time can then be shortened without lowering the measurement precision.

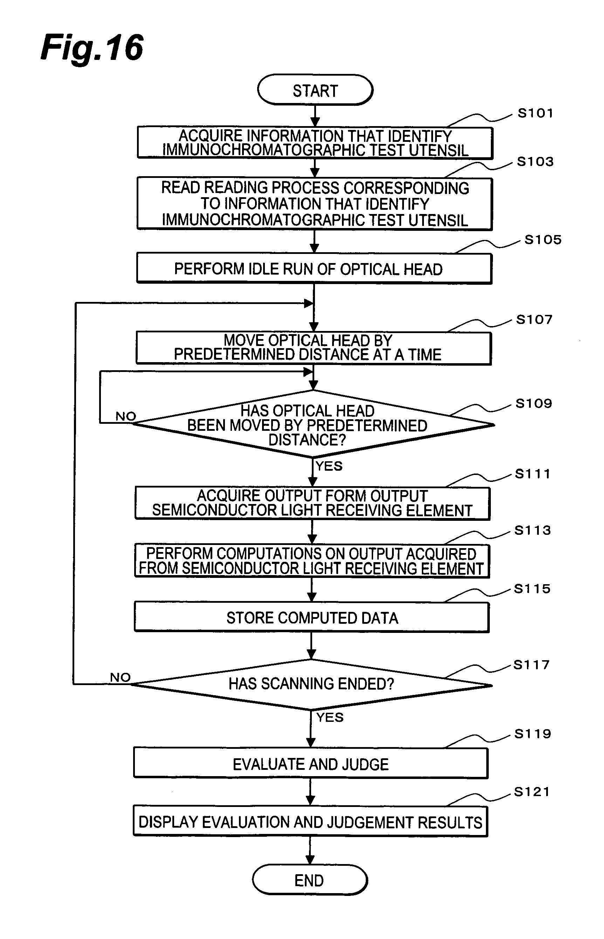

The operations of the reader 1 (controlling circuit 21) shall now be described in further detail with reference to Fig. 16. An operation example, wherein the information storage section IS1 stores just the information that identify the immunochromatographic test utensil TE1 shall be described. Fig. 16 is a flowchart for explaining the operations of the reader according to the present invention.

As shown in Fig. 12, the controlling circuit section 21 comprises a CPU 21a, EEPROM or other memory section 21b, etc. Reading processes that respectively correspond to various types of the immunochromatographic test utensils are stored in memory section 21b. Each reading process includes an operation process and a measurement process. For example, when the cartridge CA1 holding the immunochromatographic test utensil TE1 is inserted from the insertion slot 17, the connecting terminal section CT1 of the cartridge CA1 becomes connected to the connecting terminal section 39.

First, when the connecting terminal section CT1 and the connecting terminal section 39 are connected, the controlling circuit section 21 (CPU 21a) acquires the information that identify the immunochromatographic test utensil TE1 from the information storage section IS1 of the cartridge CA1 (S101) and reads the reading process (operation process and measurement process), corresponding to the information that identifies the immunochromatographic test utensil TE1, from the memory section 21b (S103). When the cartridge CA2 holding the immunochromatographic test utensil TE2 is inserted from the insertion slot 17, the connecting terminal section CT2 becomes connected to the connecting terminal section 39, and then the controlling circuit section 21 (CPU 21a) acquires the information that identify the immunochromatographic test utensil TE2 from the information storage section IS2 of the cartridge CA2 and reads the reading process (operation process and measurement process), corresponding to the information identifying the immunochromatographic test utensil TE2, from the memory section 21b.

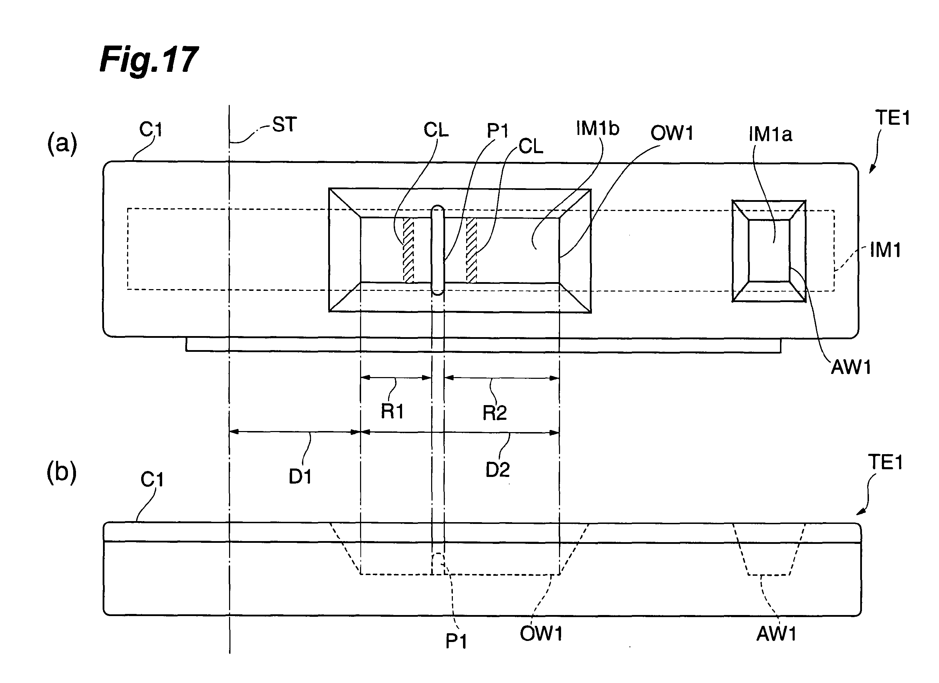

The immunochromatographic test utensil inspection (reading of colored regions) is performed by the optical head 35 scanning the observation window of the immunochromatographic test utensil, and in order to accommodate for the immunochromatographic test utensils with various shapes, the scanning range is made longer than the actual observation window. Thus the scanning speed (moving speed of the optical head 35) is made variable and colored lines CL are read upon scanning (moving) the optical head 35 at a high speed in the idle run period, when the optical head 35 is not positioned above the observation window, that is, in the period when the measuring light is illuminated on the casing C1 or C2 itself. By then scanning (moving) the optical head 35 at a low speed in the measuring period, in which measuring light is illuminated through the observation window, the measurement time is shortened. Thus as shown in Fig. 17, the operation process is set according to an idle run distance D1 from a scanning starting position ST to the end of the observation window OW1, a length (scanning distance) D2 of the observation window OW1, the positions and number of the colored lines CL, and the existence of blocking objects (partition PI) inside the observation window OW1 of each immunochromatographic test utensil (immunochromatographic test utensil TE1 is shown in Fig. 17). The operation process is thus a process of varying the scanning speed according to the position of the optical head 35. The zones R1 and R2 are also set in accordance with the positions and number of the colored lines CL.

Though the operation process is thus determined according to the shape of the immunochromatographic test utensil and especially the shape of the observation window, the measurement process is determined by the immunochromatographic test strip held by the immunochromatographic test utensil. In an immunochromatographic method, a sample is evaluated by the appearance of the colored lines CL, and a time of approximately 10 to 30 minutes after the dropping of the sample is required to obtain accurate coloring and this time also differs according to the immunochromatographic test strip. First, a waiting time to the start of scanning from the point at which the cartridge is inserted into the reader 1 is set.

Though the measurement is started after the elapse of the waiting time, the improved measurement precision for accurate reading of pale coloration may be required or reduction of the measurement time may be required in some cases. The number of times of data accumulation, operating time, etc., for the data acquisition process are thus set.

When the scanning is performed, the CPU 21a acquires data based on the output of the light receiving element 53, and for this process, the zones are set according to the positions of the colored lines CL and the variation of data within these zones are observed. Though these zones are set by the above operation process, since the significance of the data in each zone differs according to the immunochromatographic test strip, the zones are set in the measurement process. At the same time, methods of performing computations on the data obtained and judgment methods are also set in the measurement process. The data computation methods in the measurement process include those for computing average values, smoothing the output from the light receiving element 53, etc. Judgment methods in the measurement process include the computing of peak values, the computing of absorbance values, etc.

Referring again to Fig. 16, the controlling circuit section 21 (CPU 21a) starts an inspection based on the reading process (operation process and measurement process) that has been read. First, the controlling circuit section 21 makes the optical head 35 perform the idle run (movement) at high speed across just the idle run distance that was set in the operation process (S105). After the optical head 35 has been moved by just idle run distance D1, the controlling circuit section 21 (CPU 21a) makes the optical head 35 be moved at the low speed. In the present embodiment, the controlling circuit section 21 (CPU 21a) makes the optical head 35 be moved a predetermined distance (step) at a time (S107) and then judges whether movement by just the predetermined distance has been accomplished (S109). Upon judging that the movement has been accomplished, the controlling circuit section 21 (CPU 21a) acquires the output from the light receiving element 53 (S111). Thereafter, the controlling circuit section 21 (CPU 21a) performs computations on the output acquired from the light receiving element 53 based on the data computation methods of the measurement process (S113) and stores the computed data in the memory section 21b (S115). The computed data may also be outputted to an external information processing apparatus from the external output terminal 15.

Then based on whether or not the optical head 35 has been moved by just scanning distance D2, the controlling circuit section 21 (CPU 21a) judges whether or not scanning has ended (S117). If it is judged that scanning has ended, the controlling circuit section 21 (CPU 21a) reads, evaluates, and judges the data stored in the memory section 21b based on the data computing method of the measurement process (S119). The controlling circuit section 21 (CPU 21a) then displays the evaluation and judgment results on the display 7 (S121). These evaluation and judgment results may also be output to an external apparatus from the external output terminal 15.

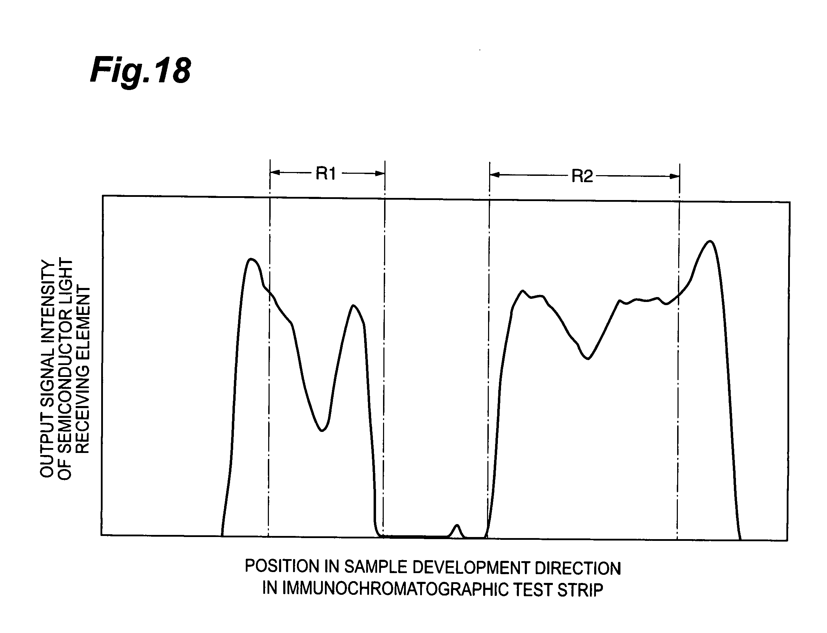

Examples of the evaluation and judgment results include a measuring light absorbance profile (see Fig. 18) that is prepared based on the data (detection signals from the light receiving element 53) stored in the memory section 21b, the output signal intensities To of the reflected light from colored lines CL that have undergone coloration, the output signal intensities Ti of the light reflected from portions that have not undergone coloration, the absorbance ABS (ABS = logTi/To) of colored lines CL of the immunochromatographic test strip IM1 or IM2 that have undergone coloration, etc. By referencing a calibration curve diagram prepared in advance, the total amounts (concentrations) of the antigens (or antibodies) contained in the sample are determined according to the computed absorbance ABS.

As described above, with the present embodiment, the cartridge CA1 or CA2, holding the immunochromatographic test utensil TE1 or TE2 (casing C1 or C2) in the recess H1 or H2 that serves as a holding section, is introduced to the position of the reader 1 at which measuring light is illuminated onto the immunochromatographic test strip IM1 or IM2. After measurement, the cartridge CA1 or CA2, holding the immunochromatographic test utensil TE1 or TE2, is taken out from the reader 1. Thus, for example, by preparing the cartridges CA1 and CA2 that accommodate a plurality of the immunochromatographic test utensils TE1 and TE2 that differ in external shape, measurements can be made in accordance with the immunochromatographic test utensils TE1 and TE2 that differ in external shape. Also, since the cartridge CA1 or CA2 has an information storage section IS1 or IS2, the immunochromatographic test utensil TE1 or TE2 and the information concerning the immunochromatographic test TE1 or TE2 can be managed readily in associated form.

The connecting terminal sections CT1 and CT2 are provided for sending the information concerning the immunochromatographic test utensils TE1 and TE2 stored in the information storage sections IS1 and IS2 to the reader 1. The information concerning the immunochromatographic test utensils TE1 and TE2 that are stored in the information storage sections IS1 and IS2 can thus be sent to the reader 1.

The cartridges CA1 and CA2 are provided with the labels S1 to S4 for identifying the immunochromatographic test utensils TE1 and TE2. The immunochromatographic test utensils TE1 and TE2 to be held by the cartridges CA1 and CA2 can thus be visually identified accurately by a person in charge of inspection, etc.

Though in regard to the operations of the reader 1, an example wherein the information storage section IS1 or IS2 of the cartridge CA1 or CA2 stores just the information that identify the immunochromatographic test utensil TE1 or TE2 was described, the information storage section IS1 or IS2 of the cartridge CA1 or CA2 may be made to store the information concerning an inspection performed using the immunochromatographic test utensil TE1 or TE2, the information concerning a colored region reading process that is in accordance with the immunochromatographic test strip IM1 or IM2 included in the immunochromatographic test utensil TE1 or TE2, the information concerning a colored region reading process that is in accordance with the casing C1 or C2 included in the immunochromatographic test utensil TE1 or TE2, the information concerning a method of reading by the reader 1, the information concerning methods of performing computations on data obtained by the reader 1, the information concerning the colored regions read by the reader 1, etc.

When the above-mentioned information storage section IS1 or IS2 is arranged from an information recording medium (such as an IC chip, etc.) that is large in storage capacity and enables reading and writing, an inspection system such as the following (inspection system according to the present invention) can be realized.

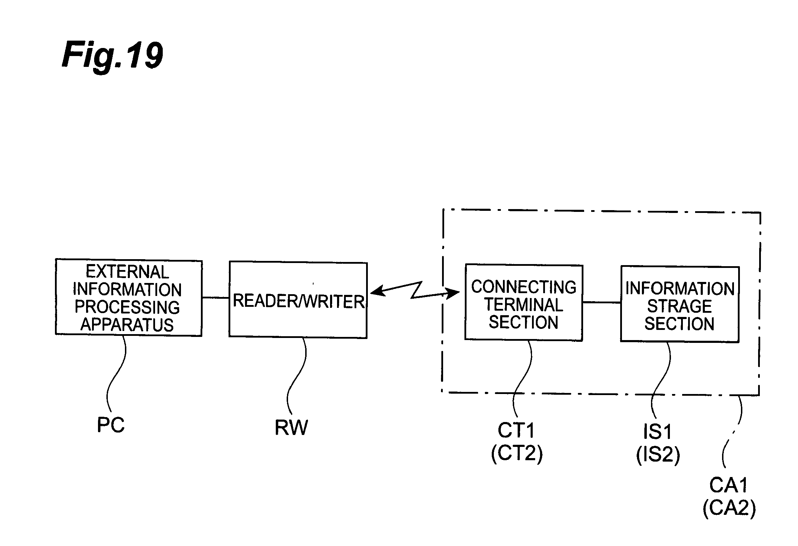

That is, as shown in Fig. 19, the information concerning an inspection performed using the immunochromatographic test utensil TE1 or TE2 are written into/deleted from the information storage section IS1 or IS2 of the cartridge CA1 or CA2 from an external information processing apparatus PC via a reader/writer RW.

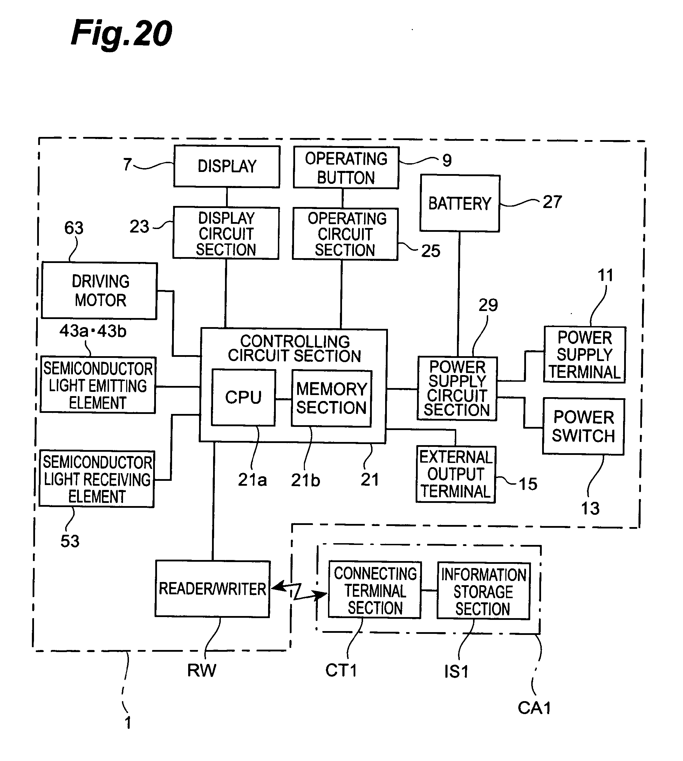

In the case where the reader 1 is equipped with reader/writer RW as shown in Fig. 20, the information concerning the colored regions read from the immunochromatographic test strip IM1 or IM2, included in the immunochromatographic test utensil TE1 or TE2 held in the inserted the cartridge CA1 or CA2, are written into/deleted from the information storage section IS1 or IS2 of the cartridge CA1 or CA2 by the reader 1 via a reader/writer RW. In this case, the immunochromatographic test utensil TE1 or TE2, held in the cartridge CA1 or CA2, and the information concerning the immunochromatographic test utensil TE1 or TE2 that are stored in the information storage section IS1 or IS2, and the information concerning the colored regions read by the reader 1 can be managed accurately in associated form.

When the information concerning the colored regions, which have been read from the immunochromatographic test strip IM1 or IM2 included in the immunochromatographic test utensil TE1 or TE2 held in the inserted cartridge CA1 or CA2, are written by the reader 1 into the information storage section IS1 or IS2 of the cartridge CA1 or CA2, the external information processing apparatus PC acquires, via reader/writer RW, the information that identify the immunochromatographic test utensil TE1 or TE2 and the information concerning the colored regions read by the reader 1 from the information storage section IS1 or IS2 of the cartridge CA1 or CA2, and the external information processing apparatus PC stores and manages these pieces of information in associated form. In this case, the information concerning the immunochromatographic test utensil TE1 or TE2 stored in the information storage section IS1 or IS2 and the information concerning the colored regions read by the reader 1 from the immunochromatographic test strip IM1 or IM2 included in the immunochromatographic test utensil TE1 or TE2 held in the inserted cartridge CA1 or CA2 can be managed accurately in associated form. The information concerning the immunochromatographic test utensil TE1 or TE2 that are stored by the external information processing apparatus PC or the information concerning the colored regions read by the reader 1 may be printed out using a printer.

The information concerning the reading processes of the respective colored regions, the information concerning the method of reading by the reader 1, or the information concerning the methods of performing computations on the data obtained by the reader 1 are stored in the information storage section IS1 or IS2 of the cartridge CA1 or CA2, the reader 1 (controlling circuit section 21) acquires these pieces of information via reader/writer RW, and determines the reading process. In this case, the memory capacity of memory section 21b, included in the controlling circuit section 21, can be reduced.

The present invention is not restricted to the above-described embodiment. For example, a contacting method or a non-contacting method may be employed for the sending and receiving of the information concerning the immunochromatographic test utensil TE1 or TE2 between the cartridge CA1 or CA2 (information storage section IS1 or IS2) and the reader 1 (controlling circuit section 21) and the sending and receiving of the information concerning the immunochromatographic test utensil TE1 or TE2 between the cartridge CA1 or CA2 (information storage section IS1 or IS2) and the external information processing apparatus PC.

From the invention thus described, it will be obvious that the embodiments of the invention may be varied in many ways. Such variations are not to be regarded as a departure from the spirit and scope of the invention, and all such modifications as would be obvious to one skilled in the art are intended for inclusion within the scope of the following claims.

## Claims

1. A cartridge, which can be detachably attached to an immunochromatographic test reader with a structure that illuminates measuring light onto an immunochromatographic test strip included in an immunochromatographic test utensil and that detects light arising from said immunochromatographic test strip as a result of illumination of the measuring light, comprising:
a holding section for holding said immunochromatographic test utensil;
information storage means for storing information concerning said immunochromatographic test utensil; and
a structure for introducing said immunochromatographic test utensil to a position where the measuring light is illuminated onto said immunochromatographic test strip included in said immunochromatographic test utensil.

2. A cartridge according to claim 1, wherein the information concerning said immunochromatographic test utensil includes information concerning an inspection using said immunochromatographic test utensil.

3. A cartridge according to claim 1, wherein the information concerning said immunochromatographic test utensil includes information that identifies said immunochromatographic test utensil.

4. A cartridge according to claim 1, wherein the information concerning said immunochromatographic test utensil includes information concerning a colored region reading process corresponding to said immunochromatographic test strip included in said immunochromatographic test utensil.

5. A cartridge according to claim 1, wherein said immunochromatographic test utensil includes a casing that holds said immunochromatographic test strip, and
wherein the information concerning said immunochromatographic test utensil includes information concerning a colored region reading process corresponding to said casing included in said immunochromatographic test utensil.

6. A cartridge according to claim 1, wherein the information concerning said immunochromatographic test utensil includes information concerning a method of reading by said reader.

7. A cartridge according to claim 1, wherein the information concerning said immunochromatographic test utensil includes information concerning methods of performing computations on data obtained by said reader.

8. A cartridge according to claim 1, wherein the information concerning said immunochromatographic test utensil includes information concerning colored regions read by said reader.

9. A cartridge according to claim 1, further comprising:
information sending means, sending the information concerning said immunochromatographic test utensil, stored in said information storage means, to said reader.

10. A cartridge according to claim 1, further comprising:
a label for identifying said immunochromatographic test utensil.

11. A cartridge according to claim 1, wherein said holding section comprises a recess with a shape corresponding to the outer shape of said immunochromatographic test utensil.

12. A cartridge according to claim 1, further comprising:
a first surface facing said immunochromatographic test utensil held in said holding section;
a second surface opposing said first surface; and
a through hole, arranged at a portion of said holding section that overlaps with said immunochromatographic test utensil, communicating said first surface and said second surface.

13. An immunochromatographic test reader, with a structure that illuminates measuring light onto an immunochromatographic test strip included in an immunochromatographic test utensil and that detects light arising from said immunochromatographic test strip as a result of illumination of the measuring light, comprising:
a structure for holding a cartridge according to any one of claims 1 to 12 at a position where the measuring light is illuminated onto said immunochromatographic test strip; and
information acquiring means for acquiring information concerning said immunochromatographic test utensil stored in said information storage means, in the state in which said cartridge is mounted.

14. An immunochromatographic test reader according to claim 13, further comprising:
an illuminating optical system illuminating the measuring light onto said immunochromatographic test strip; and
a detecting optical system detecting a reflected light from said immunochromatographic test strip resulting from the illumination of the measuring light by said illuminating optical system;
wherein said holding section of said cartridge includes a recess of a shape corresponding to the outer shape of said immunochromatographic test utensil, and said recess has a depth such that the optical axis of said illuminating optical system and the optical axis of said detecting optical system intersect at a surface of said immunochromatographic test strip included in said immunochromatographic test utensil in the state of being fitted into said recess.

15. An immunochromatographic test reader according to claim 14, wherein said cartridge comprises:
a first surface facing said immunochromatographic test utensil held in said holding section;
a second surface opposing said first surface; and
a through hole, arranged at a portion of said holding section that overlaps with said immunochromatographic test utensil, communicating said first surface and said second surface;
wherein said reader further comprises a structure for exposing said through hole, provided in said cartridge, to the exterior of said reader in the state wherein said cartridge is introduced inside said reader.

16. An immunochromatographic test reader according to claim 13, further comprising:
an illuminating optical system illuminating the measuring light onto said immunochromatographic test strip;
a detecting optical system detecting a reflected light from said immunochromatographic test strip resulting from the illumination of the measuring light by said illuminating optical system;
an optical head on which said illuminating optical system and said detecting optical system are provided; and
a scanning mechanism moving said cartridge introduced in said reader and said optical head in a relative manner.

17. An immunochromatographic test reader according to claim 16, wherein said scanning mechanism moves said cartridge and said optical head relative to each other at a first speed during a period, in which the measuring light is illuminated onto said immunochromatographic test strip via a window provided in said immunochromatographic test utensil, and moves said cartridge and said optical head relative to each other at a second speed higher than the first speed during a period, in which the measuring light is illuminated onto portions besides the window provided in said immunochromatographic test utensil.

18. An immunochromatographic test strip inspection system comprising:
an immunochromatographic test reader according to any one of claims 13 to 17; and
means that stores, in associated form, information concerning said immunochromatographic test utensil, which are stored in said information storage means, and information concerning colored regions read by said reader.

19. An inspection system according to claim 18, wherein the information concerning said colored regions read by said reader are sent to said cartridge and stored in said information storage means of said cartridge.
